# EUROPEAN PATENT APPLICATION

(11) **EP 4 364 730 A1**
(43) Date of publication of application: **08.05.2024**
(21) Application number: 23206732.2
(22) Date of filing: 30.10.2023
(51) Int. Cl.: A61K 31/015, A61K 31/221, A61P 25/02, A61P 29/00, A23G 1/00

(54) **PHARMACEUTICAL COMPOSITION COMPRISING L-ACETYL CARNITINE AND BETA-CARYOPHYLLENE**

(30) Priority: 02.11.2022 IT 202200022437
(71) Applicant: Chiesi Italia S.p.A., 43122 Parma (PR) (IT)
(72) Inventor: RINALDI, Laura, 43122 PARMA (PR) (IT); BOCCHINO, Boris, 43122 PARMA (PR) (IT); PALEARI, Davide, 43122 PARMA (PR) (IT); DAGLIA, Maria, 80131 NAPOLI (IT); DI MINNO, Alessandro, 80131 NAPOLI (IT)
(74) Representative: Bianchetti & Minoja with Trevisan & Cuonzo IPS SRL

(57) **Abstract**

The present invention concerns a combination for use in humans comprising L-acetyl carnitine and beta-caryophyllene for the prevention and/or treatment of inflammation, pain and/or pain-correlated diseases.

## Description

### TECHNICAL FIELD OF THE INVENTION

The present invention concerns a combination for use in humans comprising L-acetyl carnitine and beta-caryophyllene for the prevention and/or treatment of inflammation, pain and/or pain-correlated diseases.

### BACKGROUND OF THE INVENTION

Pain has been described as an unpleasant sensation that occurs as a result of injury to the body or as a manifestation of a disease state.

Pain can be classified in many ways: based on its duration as acute or chronic pain; on its severity as mild, moderate or severe; on its underlying cause as nociceptive, inflammatory or neuropathic.

Acute pain is characterized by early onset with a relatively short duration, lasting no more than days or weeks. Acute pain is seen with trauma, surgical interventions and pain caused by certain diseases, such as tumours that invade organs.

Chronic pain is defined as pain persisting for more than one month beyond the usual course of an acute illness or the time required for an injury to heal, pain associated with a chronic pathologic process, or pain recurring at intervals of months or years.

Nociceptive pain results from a direct tissue damage deriving for example from surgical incisions, bone fractures, metastatic cancer or joint diseases, such as osteoarthritis and rheumatoid arthritis.

Inflammatory pain involves the release of mediators, which sensitize peripheral nociceptors. Nociceptors sensitization plays an important role in central sensitization and clinical pain states such as: i) an increased response to a noxious stimulation (hyperalgesia) ii) a painful response to a normally innocuous stimulus (allodynia).

Neuropathic pain occurs because of damage to, or dysfunction of, the nervous system.

Inflammation and neuropathy are the two major pathophysiological changes underlying different chronic pain mechanisms.

Chronic inflammatory pain results from peripheral tissue injury produced by infection or trauma, or diseases with an autoimmune component, such as arthritis.

Chronic neuropathic or neurogenic pain results from nerve injury associated with trauma, radiation damage, surgery, crushed limbs or amputation, and diseases such as herpes zoster, multiple sclerosis, arthritis, and diabetes, or from cancer chemotherapy. Moreover, it occurs in situations where a nerve is trapped and/or compressed (e.g. carpal tunnel syndrome, herniated disc, cervicobrachial pain, neck pain, low back pain).

Diabetic neuropathy is the most common neuropathic pain syndrome.

Thanks to its anti-inflammatory and antioxidant properties, L-acetyl carnitine (LAC) is used for the prevention and/or treatment of inflammation, pain and pain-correlated diseases, such as neuropathies.

For example, US 4751242 describes the use of LAC for the treatment of neuropathic pain in patients with peripheral neuropathy of various origins, including diabetic peripheral neuropathy. WO 02096409 discloses the use of LAC for early administration in relation to painful events.

WO2006114372 concerns the use of LAC for the prevention of painful peripheral neuropathy in patients suffering from type 2 diabetes.

On the other hand, the evidence about its efficacy requires further investigation to be confirmed.

Moreover, to improve its efficacy, LAC has been associated with other substances, for example palmitoylethanolamide (EP 2921167; Paris S et al Minerva Med 2021, 112(4):492-499) alpha-lipoic acid and curcumin (WO 2016/157053), vitamins and vegetable extracts (Evans M et al Nutrients 2020, 12, 1831).

The management of pain is challenging. The first option to relieve pain is the use of symptomatic drugs, which generally have a good and rapid efficacy although, in some cases, they possess limited efficacy and considerable side effects. Growing evidence support the use of LAC and many efforts have been spent to identify combinations of LAC with different compounds to increase its effectiveness, although to date further studies are required. Therefore, there is still the need of tolerated analgesic therapy, based on the use of LAC, for the prevention and/or treatment of inflammation, pain and pain-correlated diseases.

The solution is provided by the combination of the present invention.

### SUMMARY OF THE INVENTION

A first aspect of the present invention is a combination comprising L-acetyl carnitine (hereinafter also indicated as LAC) or a pharmaceutically acceptable salt thereof and beta-caryophyllene (hereinafter also indicated as BCP).

The molar ratio between L-acetyl carnitine or a pharmaceutically acceptable salt thereof and beta-caryophyllene is comprised between 1:2 and 20:1.

The combination may be in the form of a fixed combination or loose combination, preferably, as a fixed combination.

In a second aspect, the invention provides a composition comprising a combination containing L-acetyl carnitine or a pharmaceutically acceptable salt thereof and beta-caryophyllene, together with one or more pharmaceutically or physiologically acceptable excipient and/or additive.

In a third aspect, the invention is directed to a kit comprising: a) L-acetyl carnitine or a pharmaceutically acceptable salt thereof in a first dosage unit form b) beta-caryophyllene in a second dosage unit form; and optionally c) a unique packaging container for said first and second dosage forms.

In a fourth aspect, the invention is directed to a combination comprising L-acetyl carnitine or a pharmaceutically acceptable salt thereof, and beta-caryophyllene for use as a medicament.

In a fifth aspect, the invention is directed to L-acetyl carnitine or a pharmaceutically acceptable salt thereof in combination with beta-caryophyllene for use in the prevention or treatment of inflammation, pain and pain-correlated diseases, such as neuropathies, inflammatory pain and fibromylagia.

The invention is also directed to the use of L-acetyl carnitine or a pharmaceutically acceptable salt thereof in combination with beta-caryophyllene for the preparation of a medicament for the prevention and/or treatment of inflammation, pain and pain-correlated diseases, such as neuropathies, inflammatory pain and fibromyalgia.

Further, the invention is directed to a method of prevention and/or treatment inflammation, pain, and pain-correlated diseases, such as neuropathies, inflammatory pain and fibromyalgia in a subject in need thereof, comprising administering an effective amount of a combination comprising L-acetyl carnitine or a pharmaceutically acceptable salt thereof and beta-caryophyllene.

### DEFINITIONS

As used herein, the term "medicament" refers to any composition providing health benefits, preventing and/or treating diseases including medicines, nutraceuticals and/or food supplements used for health purposes.

The term "fixed combination" means a combination wherein the active substances are in a fixed quantitative ratio in a single dosage form.

The term "pharmaceutically or physiologically acceptable" refers to excipients, carriers, additives or vehicles that do not produce an allergic or unwanted reaction when administered to a human being.

The term "synergistic" means that the activity of the two compounds results in an effect which is more than what would be expected to result from the sum by summing their respective individual activities in a given assay.

The term "prevention" means an approach aimed at reducing the risk of onset of a disease.

The term "treatment" means an approach aimed at obtaining beneficial results, including clinical results. Beneficial results can include, but are not limited to, alleviation or amelioration of one or more symptoms or conditions, diminishment of the extent of the disease, stabilization (i. e. not worsening) of the state of the disease, preventing the spread of the disease, delay or slowing of the disease progression, amelioration or palliation of the disease state, and remission (whether partial or total), whether detectable or undetectable.

The term "L-acetyl carnitine", refers to the R-isomer of acetyl-carnitine ((3*R*)-3 acetyloxy-4-(trimethylazaniumyl)butanoate), also known as O-Acetyl-L-carnitine or Levocarnitine acetyl.

The term "beta-caryophyllene" (referred to hereinafter also as BCP) refers to means the (-) enantiomer form. Beta-caryophyllene (chemical name (1*R*,4*E*,9*S*)-4,11,11-trimethyl-8-methylidenebicyclo[7.2.0]undec-4-ene) has the stereocenter adjacent to the exocyclic double bond of S configuration while the other remaining stereocenter has R configuration. It is the most common form of beta-caryophyllene, present in many essential oils. Its (+) enantiomer has minor anti-inflammatory properties.

### BRIEF DESCRIPTION OF THE FIGURES

**Figure 1** shows cellular viability of the LAC and BCP combination in the concentration range reported in Table 1.
**Figure 2** shows percentage of inhibition of IL-8 released in the supernatants of cells treated with LAC, BCP alone and in combination.
**Figure 3** shows percentage of inhibition of IL-6 released in the supernatants of cells treated with LAC, BCP alone and in combination.
**Figure 4** shows gene expression fold-change values derived from qPCR data. The changes in expression of IL-6 in HMC3 cell line data were normalized to β-actin (ACTB).
**Figure 5** shows gene expression fold-change values derived from qPCR data. The changes in expression of IL-8 (also called CXCL-8) in HMC3 cell line data were normalized to β-actin (ACTB).
**Figure 6** shows percentage of ROS production following treatment of BCP, LAC, and in combination (Mix 1-9). CTR P.I.C.: positive control. CTR DCF: control with dichlorofluorescein.

### DETAILED DESCRIPTION OF THE INVENTION

The inventors have surprisingly found that the combination of L-acetyl carnitine and beta-caryophyllene provides a synergistic effect, particularly on some markers of inflammation and/or pain.

Upon cell treatment with the two active ingredients together a synergistic effect on the interleukine-6 (IL-6) and interleukine-8 (IL-8) markers has been observed.

Furthermore, the combination, in molar ratios within the claimed range, induces a reduction on a marker of the oxidative stress, i.e. ROS.

The combination of the invention is therefore suitable for the prevention and/or treatment of inflammation, pain and pain-correlated diseases, such as neuropathies and inflammatory pain.

Therefore, the invention is directed to a combination comprising L-acetyl carnitine or a pharmaceutically acceptable salt thereof and beta-caryophyllene, preferably LAC and BCP are present in a fixed combination.

L-acetyl carnitine is a commercially available product, for example from Sigma Aldrich Co (Missouri, USA). Advantageously its optical purity is higher than 95%, preferably higher than 98%, more preferably higher than 99%.

Advantageously L-acetyl carnitine is used in form of pharmaceutically acceptable salt. Examples of such salts include hydrochloride, chloride, bromide, orotate, acid aspartate, acid citrate, magnesium citrate, acid phosphate, fumarate and acid fumarate, magnesium fumarate, lactate, maleate, acid maleate, mucate, acid oxalate, pamoate, acid pamoate, acid sulphate, glucose phosphate, tartrate, acid tartrate, magnesium tartrate, 2-amino ethane sulphonate, magnesium 2-amino ethanesulphonate, choline tartrate and trichloroacetate.

In a preferred embodiment, L-acetyl carnitine is used as hydrochloride salt.

L-acetyl carnitine may be used as non-micronized or micronized powder.

Beta-caryophyllene is a naturally occurring terpene found in black pepper and clove oil, as well as rosemary, oregano, cinnamon, basil, black caraway, hops, and essential oils.

In some embodiments, beta-caryophyllene may be used in the pure form, which is commercially available, for example from Sigma Aldrich Co (Missouri, USA). Advantageously, its optical purity is higher than 95%, preferably higher than 98%, more preferably higher than 99%.

In other embodiments, beta-caryophyllene may be used as a natural extract, deriving from black pepper (*Piper nigrum* L.), cloves (*Syzygium aromaticum* or *Eugenia caryophyllus)*, lemon balm (*Melissa officinalis*), rosemary (*Rosmarinus officinalis*), hop (*Humulus lupulus*), cinnamon (*Cinnamomum verum*) and origan (*Origanum vulgare*), preferably black pepper.

For example, a fluid extract of black pepper with a high standardized content of β-caryophyllene is commercially available as PipeNig^{®}-FL from Biosfered S.r.L. (Torino, Italy).

Another useful source of beta-caryophyllene is copaiba oil (*Copaifera officinalis*). It is for example available in pharma grade from A.G Industries, India.

Based on the natural extract title, the skilled person would calculate the correct amount to be added in order to achieve the desired amount of beta-caryophyllene.

The skilled person could easily determine the extract title by titration, if necessary.

Advantageously, the combination of the invention may comprise L-acetyl carnitine and beta-caryophyllene in a molar ratio comprised between 1:2 and 20:1, preferably between 1:1 and 15:1, more preferably between 2:1 and 10:1.

Since the molecular weight of LAC as a base is similar to that of BCP (203.24 vs. 204.36), the molar ratio approximatively corresponds to the weight ratio.

In one embodiment, when the combination of the invention is administered as a nutraceutical or food supplement, the molar ratio is comprised between 1:1.5 and 12:1 and beta-caryophyllene is utilised as natural extract only.

In an alternative embodiment, when the combination of the invention is administered as medicine in a pharmaceutical formulation, the molar ratio is comprised between 1:2 and 10:1 and beta-caryophyllene may be utilised both as natural extract and in the pure form.

The composition of the invention may further comprise at least one additional active ingredient such as antioxidants, anti-inflammatory agents, and/or vitamins.

Advantageously, antioxidants may be selected from the group comprising polyphenols, alpha-lipoic acid (also known as thioctic acid), vitamins, coenzyme Q10, and glutathione.

When the antioxidant compound is or comprises a polyphenol, it is preferably selected from polydatin, resveratrol, luteolin, quercetin and rutin.

Suitable compounds with anti-inflammatory activity include for example palmitoylethanolamide and *Boswellia serrata* extracts.

Vitamins are preferably selected from vitamins of the B group, more preferably vitamin B6 and vitamin E.

In a preferred embodiment of the invention, the antioxidant agent is alpha-lipoic acid. In fact, said acid, due to its strong antioxidant properties, may contribute to reduce inflammation and to preserve β-caryophyllene against oxidative stress.

The invention is also directed to compositions comprising the combination of the invention and pharmaceutically or physiologically acceptable excipients and/or additives.

The combination and compositions according to the invention may be formulated for oral, buccal, or rectal administration, preferably for oral administration.

For the oral administration, the compositions may be, for example, in the form of tablets or capsules prepared by conventional methods with pharmaceutically or physiologically acceptable excipients, such as binders (e.g., polyvinylpyrrolidone or hydroxypropyl methylcellulose); bulking agents (e.g., calcium carbonate, dibasic calcium phosphate, tribasic calcium phosphate, calcium sulfate, microcrystalline cellulose, powdered cellulose, dextrans, dextrin, dextrose, fructose, kaolin, lactose, mannitol); lubricants such as magnesium stearate, calcium stearate, stearic acid, sodium stearyl fumarate and/glidants (e.g. colloidal silicon dioxide, starch and talc).

For the oral administration, the compositions may also be formulated as liquid preparations for oral administration, for example, in the form of solutions, syrups or suspensions, or they can be in the form of lyophilized products to be reconstituted, before their use, with water or other suitable vehicles. Such liquid preparations can be prepared by conventional methods with pharmaceutically or physiologically acceptable additives, such as suspending agents (e.g., sorbitol syrup, cellulose derivatives, or edible hydrogenated fats); emulsifying agents (e.g., lecithin or acacia); non-aqueous vehicles (e.g., almond oil, oily esters, ethyl alcohol, or fractionated plant-based oils); and preservatives (e.g., methyl- or propyl-p-hydroxybenzoates or sorbic acid). The compositions may also suitably contain flavors, colorants, and sweeteners.

For the buccal administration, the compositions can be in the form of tablets or lozenges formulated by conventional methods, suitable for an absorption by the buccal mucosae. Typical buccal formulations are the tablets for sub-lingual administration. According to the invention, the compositions may also be formulated in rectal compositions, such as suppositories or retention enema, for example containing the base components typical of suppositories, such as cocoa butter or other glycerides.

The compositions described above can be prepared according to conventional methods, such as those described in Remington's Pharmaceutical Sciences Handbook, Mack Pub. Co., N.Y., USA.

The dose of the active ingredients proposed for the administration will depend on the foreseen use.

Typically, as a nutraceutical or food supplement, for a human being (with a body weight of about 70 Kg), the dose of L-acetyl carnitine may vary from 200 mg to 3000 mg per dose unit, preferably from 400 mg to 2000 mg, more preferably from 500 and 1500 mg, while the dose of beta-caryophyllene may vary from 20 and 1500 mg, preferably from 40 mg and 800 mg, more preferably from 50 to 400 mg.

Particularly preferred doses are selected from LAC 500 mg and BCP from 50 to 100 mg; or LAC 1000 mg and BCP from 100 to 200 mg; or LAC 1500 mg and BCP from 150 to 300 mg.

When it is administered as a medicine in the form of a pharmaceutical composition, the dose of L-acetyl carnitine may vary from 200 mg and 1500 mg, preferably from 500 to 1000 mg (expressed as a base), while the dose of beta-caryophyllene may vary from 20 mg and 400 mg, preferably from 50 and 200 mg. For example, for a ratio LAC:BCP 1:1.5, the dose of LAC could be of 500 mg, while that of BCP could be 750 mg.

The dose unit can be administered, for example, 1 to 4 times/day. The dose will depend on the selected administration route.

It could be necessary to continuously vary the dosage as a function of the age and weight of the patient, and of the severity of the clinical condition to be treated. The posology, i.e. the dose and the frequency of the administration was at discretion of the physician.

The LAC/BCP combination according to the invention may be administrated as a single therapy or as a combination therapy with other nutraceuticals, food supplement, and/or medicines, such as analgesic/anti-inflammatory drugs such as NSAIDs, paracetamol, opioids, gabapentin, pregabalin, muscle relaxants, and cannabinoids.

The combination of the present invention is suitable for the prevention and/or treatment of subjects suffering of inflammation, moderate to severe, acute or chronic pain, or pain-correlated diseases for example pain caused by different nociceptive stimuli.

The combination of the present invention is suitable for the prevention and/or treatment of mixed pain of any severity, i.e. mild, moderate and severe pain as well as including both the acute and chronic conditions.

Examples of moderate to severe acute pain include pain caused by trauma, or surgical intervention such as post-operative pain, and pain caused by diseases such as cancer, AIDS, myocardial infarction and renal or biliar colic. Examples of moderate to severe chronic pain comprise diseases such as chronic inflammatory pain or chronic neuropathic pain. Said diseases may result from peripheral tissue injury produced by infection or trauma or nerve injury associated with trauma, radiation damage, surgery, crushed limbs or amputation. Chronic pain diseases may also result from diseases such as cancer, non-malignant progressive disease (e.g., AIDS, sickle cell anemia, hemophilia, and connective tissue diseases), non-progressive or slowly progressive diseases (e.g., severe osteoporosis, gout, post-herpetic neuralgia, painful polyneuropathy, reflex sympathetic dystrophy), and idiopathic syndromes (e.g., fibromyalgia, atypical facial pain, chronic pelvic pain of unknown etiology). The combination of the invention may also be used in the prevention and/or treatment of neuropathic pain, also inhibiting the development of tolerance, and/or physical dependence on a narcotic analgesic substance.

Examples of neuropathic pain include diseases in which pain is due to a trapped and/or compressed nerve (e.g. carpal tunnel syndrome, herniated disc, cervicobrachial pain, neck pain, low back pain).

The invention is further illustrated in detail by the following Examples.

### EXAMPLE 1 - Studies with HMC3 cell line: experimental part

The following experiments were performed on the HMC3 cell line.

Thus, this cell line (HMC3) was obtained from American Type Culture Collection (ATCC) and all reagents requested to perform the experiments with these types of human cells were purchased.

### Materials

- L-acetyl carnitine HCl (hereinafter LAC) purchased from Sigma-Aldrich (A6706))
- Beta-caryophyllene (hereinafter BCP) purchased from Sigma-Aldrich (code (W225299)
- Microglial cell line, HMC3 (*Homo sapiens*, human) (CRL-3304^{™}), ATCC;
- DMEM Medium, Life Technologies Italia/Gibco^{™};
- Human IL-8 ELISAPRO Kit (3560-1HP-1);
- Human IL-6 ELISAPRO Kit (3460-1HP-1);
- Human IL-4 ELISAPRO Kit (3410-1HP-2);
- Human IL-10 ELISAPRO Kit (3430-1HP-2);
- Polyinosinic-polycytidylic acid (poly(I: C)), Invivogen;
- Hs_IL6_1_SG QuantiTect Primer Assay (QT00083720) ;
- Hs_CXCL8_1_SG QuantiTect Primer Assay (QT00000322) ;
- Hs_IL10_1_SG QuantiTect Primer Assay (QT00041685) ;
- Hs_IL4_1_SG QuantiTect Primer Assay (QT00012565) ;

### Methods

### HMC3 cell line

The American Type Culture Collection's HMC3 (CRL-3304) cell line was grown in the MEM growth medium supplemented with 10% fetal bovine serum (FBS), 100 U/mL penicillin, and 100 µg/mL streptomycin (complete medium). The cells were kept at 37 °C in a 5% CO₂ incubator and grown up to 100% confluence before use.

### Cell viability test (MTT assay)

The cell viability assays performed on the HMC3 cell line were first carried out.

In particular, regarding BCP, cells were treated with this substance at concentrations ranging from 0.039 to 100 µM (9 concentrations).

Regarding LAC, cells were treated with this substance at concentrations ranging from 0.06 to 30 mM (9 concentrations).

Not treated cells, which showed 100% of cell viability, were used as negative control.

The MTT cell viability test was performed by seeding 1 × 10⁴ cells/well in a 96-multiwell and grown at 37 °C and 5% CO₂. The next day, cells were treated with different concentrations of BCP and LAC and their combinations (*Mix*). Subsequently, the supernatant was removed from each well and 3-(4,5-dimethylthiazol-2-yl)-2,5-diphenyltetrazolium bromide (MTT, Sigma-Aldrich, St. Louis, MO) was added to the final concentration of 0.5 mg/ml. Cells were incubated for 3h at 37 °C. The formazane crystals were then solubilized with 100 µL DMSO/well for 15 min and the absorbance was measured at the wavelength of 570 nm using a Multiskan^{™} GO microplate reader (Thermo Scientific Microplate Spectrophotometer).

Finally, the cytotoxicity of the substances in combination was evaluated starting from the maximum non-cytotoxic concentrations of each molecule (BCP and LAC alone) (Table 1).

**Table 1**

| | |
|---|---|
| Mix: 1-A | β-caryophyllene 100 µM; O-Acetyl-L-carnitine HCl 2.031 mM; |
| Mix: 2-B | β-caryophyllene 50 µM O-Acetyl-L-carnitine HCl 1.016 mM; |
| Mix: 3-C | β-caryophyllene 25 µM O-Acetyl-L-carnitine HCl 0.508 mM; |
| Mix: 4-D | β-caryophyllene 12.5 µM O-Acetyl-L-carnitine HCl 0.254 mM; |
| Mix: 5-E | β-caryophyllene 6.25 µM O-Acetyl-L-carnitine HCl 0.127 mM; |

To determine the activity on the inflammation markers, the following experiments were performed on:
- LAC (at 3 concentrations);
- BCP (at 3 concentrations);
- 9 Mix deriving from the combination of LAC and BCP at 3 different concentrations as reported in Table 2.

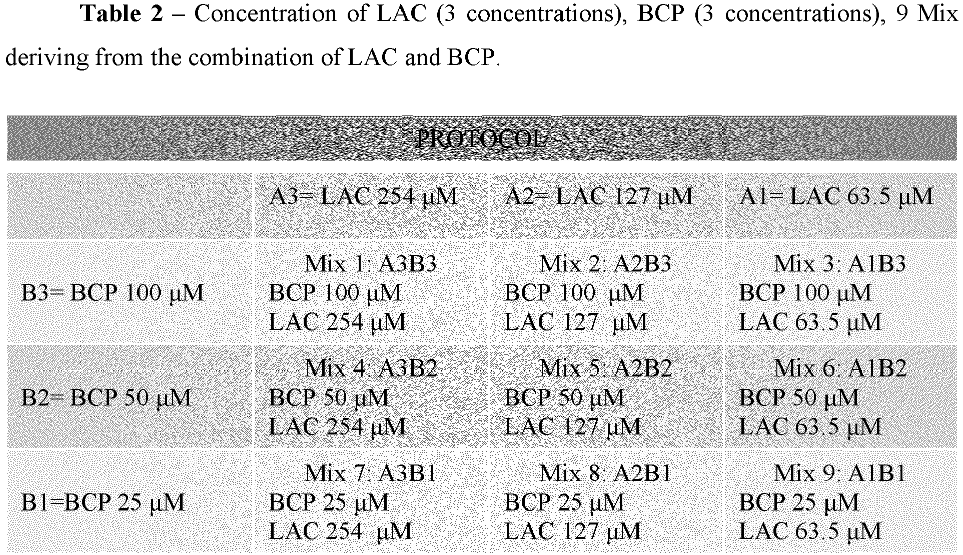

### Evaluation of pro-inflammatory cytokine levels by ELISA assay

The concentrations of IL-6, IL-8in HMC3 cell culture supernatants were evaluated through ELISA assay. All reagents in the ELISA kits have been brought to room temperature before use. Samples were not diluted (NEAT); standards with known concentrations and blank for the elaboration of the calibration curve have been prepared. The samples and standards were incubated together with the primary antibody and the secondary antibody in the multi-well plates. Therefore, the substrate of the enzyme peroxidase conjugated to the secondary antibody was added to develop a fluorescence product whose absorbance, being directly proportional to the concentration of the specific cytokine present in the samples, was evaluated at the wavelength of 450 nm.

The evaluation of the *in vitro* efficacy has been conducted using the substances alone and in combination, upon treatment of the cells as compared to the inflammatory insult induced with Poly (I:C) to determine the concentration of anti-inflammatory and immunomodulatory markers in the supernatant by ELISA assay. Finally, cell receiving no pro-inflammatory insult and cells treated with Poly (I:C) were used as negative and positive controls, respectively.

### Treatment of inflammation in HMC3 cells

The HMC3 cells were transferred into a 24-multiwell plate: for each well, 4×10⁴ cells were seeded in 1 ml of medium and incubated for 24 h. The culture medium was removed, and cells treated with the compounds alone and in combination for 3 h. A first solution of Poly I:C at a concentration of 1000 µg/mL (stock solution) was prepared by resuspending 1 mg of Poly I:C in 1 mL of PBS. Subsequently, a stock solution was prepared at a concentration of 100 µg/mL by diluting the 1:10 stock solution and 4 µL of the Poly I:C solution was then added to each well to obtain a final concentration of 4 µg/mL, for 24 h. At the end of the experiment, all the samples were collected in 1.5 mL microtubes and centrifuged (2000 rpm for 5 min, 25 °C) to induce the sedimentation of the cellular pellet. The supernatants were collected and stored at - 20 °C until the subsequent analyses by ELISA assay.

### Real-time PCR

Total RNA was extracted from HMC3 cells using the Rneasy Mini Kit (Qiagen). The quality and the quantity of RNA were checked using the Nanodrop 3.0 (Thermofisher Scientific; Munich). The reverse transcription was performed using 0.025 (microg/microL) of RNA using the QuantiTect Reverse Transcription Kit (Qiagen). The primers used in the qPCR assays were already validated and used to assess the presence of IL-6, IL-8 (CXCL8), IL-10 and IL-4 genes in human microglial cells. The qPCR was performed using the QuantiTect SYBR Green RT-PCR Kit (Qiagen) following this cycling conditions: 15s/95 °C (activation), 15s/95 °C (denaturation), 30 s/60 °C (annealing and extension) with fluorescence measurement.

The qPCR analyses were conducted with three biological and technical triplicates in the average calculation.

### Evaluation of reactive oxygen species (ROS)

For the experiment, 1×10⁴ cells/well were seeded in a 96-multiwell and grown until the next day at 37 °C at 5% CO₂. The culture medium was removed and the cells were treated with different concentrations of BCP, LAC and their combinations (Mix-1, Mix-2, Mix-3, Mix-4, Mix-5, Mix-6, Mix-7, Mix-8, Mix-9) as shown in Table 2 for 3 h. To each well, 100 µL of fresh medium containing the Poly (I:C), at the final concentration of 4 ug/mL, were added.

After 24 h of incubation, the supernatant was removed, and the cells were washed with a solution of D-PBS. The mixture of DCF (5 µM in HBSS 1% FBS) was prepared and 100 µL/well were added by incubating the cells at 37 °C for 1h. In conclusion, the plate was read at a spectrofluorometer (GloMax^{®} Explorer Multimode Microplate Reader, Promega) at an emission wavelength of 538 nm and an excitation wavelength of 485 nm. Finally, cell receiving no pro-inflammatory insult and cells treated with Poly (I:C) were used as negative and positive controls, respectively.

### Statistical analysis

Data are reported as mean ± standard deviation (SD). Statistical comparison among groups was conducted using one-way ANOVA followed by Dunnett's or Tukey's *post hoc* test for multiple comparison to determine significance, which was set to P < 0.05. For each concentration used in the ELISA assay a biological replicate were obtained and averaged. The statistical analyses were performed using Prism Graphpad 8 (San Diego, CA, USA).

### Determination of non-cytotoxic concentrations of BCP, LAC, and their combinations (Mix-1, Mix-2, Mix-3, Mix-4, Mix-5, Mix-6, Mix-7, Mix-8, Mix-9).

For BCP the maximum non-cytotoxic concentration was 100 µM, while For LAC the maximum non-cytotoxic concentration was 2.031 mM.

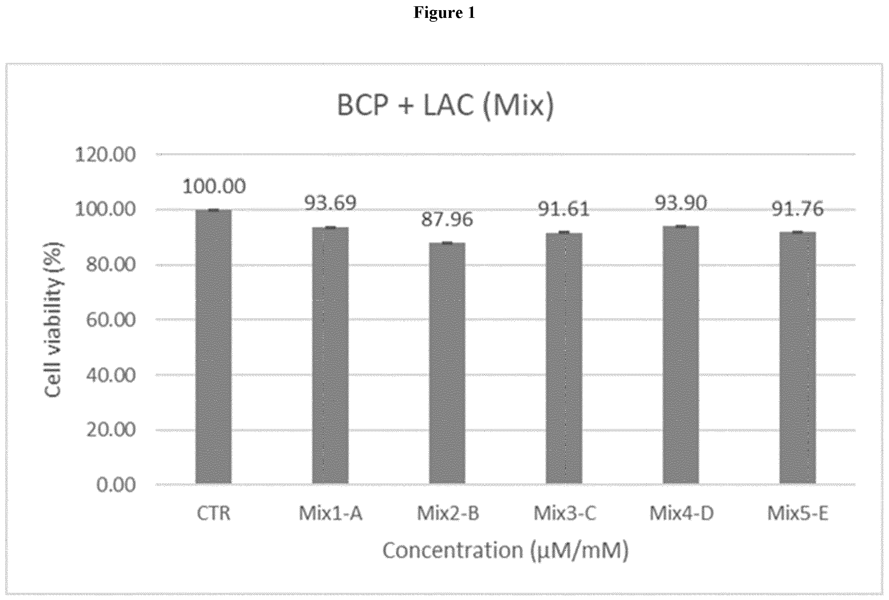

As far as the combinations of LAC and BCP are concerned, **Figure 1** shows the results obtained from the cell viability assay performed using the maximum non-cytotoxic concentrations of the two compounds in combination identified by the previous MTT assays. All the concentrations tested (Mix1-A, Mix2-B, Mix3-C, Mix4-D and Mix5-E) showed a cell viability greater than 80 %.

The experiments on the inflammation markers are performed with concentrations well below the maximal tolerable concentrations.

### EXAMPLE 2 - Studies with HMC3 cell line-treatment results.

### Effect on IL-8

Treatment with Poly (I:C) induces the production of IL-8 (positive control) and different concentration of IL-8 were released following the treatment with BCP and LAC and in combination.

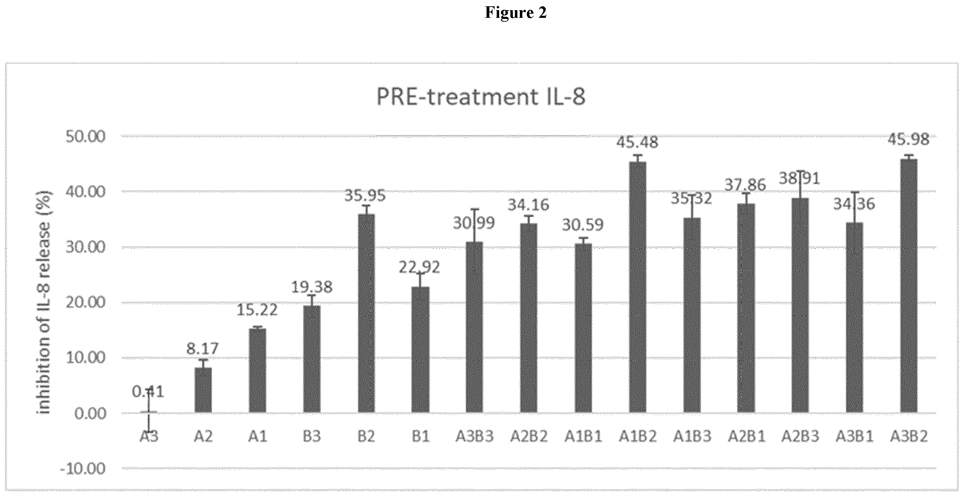

**Figure 2** shows that A3B3, A2B1, A2B3, A3B1 and A3B2 are able to reduce the concentration of IL-8, independently from the tested concentration, in comparison with the positive control, in the applied experimental conditions, indicating that these Mixes exert anti-inflammatory activity. In particular, they show an inhibition of the release of IL-8 (expressed as %) higher than the sum of the activities of the substances alone exerting synergistic activities.

Statistical analysis revealed a statistically significant difference between these Mixs and the single components (BCP and LAC) at the corresponding concentrations upon treatment as evidenced in Table 3.

**Table 3. Results of one-way Anova test (Tukey's multiple comparison) performed on BCP, LAC and their combinations.**

| **Experimental model** | **Test F** | **P-value** |
|---|---|---|
| **Mix A3B3 vs LAC 254 µM , BCP 100 µM** | 40,09 | 0.0003 |
| **Mix A2B1 vs LAC 127 µM, BCP 25 µM** | 170.9 | <0.0001 |
| **Mix A2B3 vs LAC 127 µM, BCP 100 µM** | 77.40 | <0.0001 |
| **Mix A3B1 vs LAC 254 µM, BCP 25 µM** | 51.40 | 0.0002 |
| **Mix A3B2 vs LAC 254 µM, BCP 50 µM** | 289.2 | <0.0001 |

### Effect on IL-6

Treatment with Poly (I:C) induce the production of IL-6 (positive control) and different concentrations of IL-6 were released due to the treatment with BCP and LAC, alone and in combination.

Similar to what was done previously, the activities of BCP and LAC alone, and their combinations at different concentrations (Table 2) were tested for the evaluation of the release of IL-6 in the supernatants of HMC3 cells treated with the inflammatory insult.

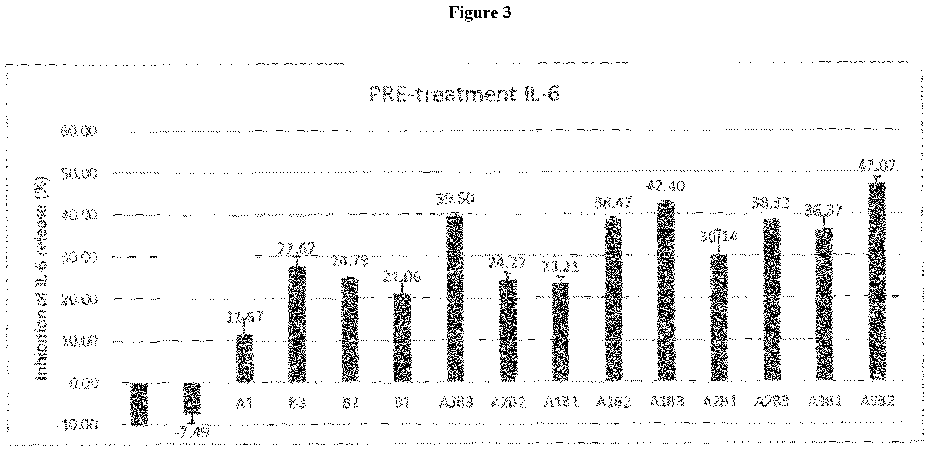

**Figure 3** shows that the same combinations of BCP and LAC (A3B3, A2B1, A2B3, A3B1, A3B2) described for the IL-8, are able to reduce the concentration of IL-6 in comparison with the positive control, in the applied experimental conditions, indicating a synergistic effect of these combinations in comparison with BCP and LAC alone.

Statistical analysis revealed a statistically significant difference between these Mixes and single components at the corresponding concentrations as evidenced by statistical parameters shown in Table 4.

**Table 4 - Results of one-way Anova test (Tukey's multiple comparison) performed on BCP, LAC and their combinations.**

| **Experimental model** | **Test F** | **P-value** |
|---|---|---|
| **Mix A3B3 vs LAC 254 µM, BCP 100 µM** | 483.9 | <0.0001 |
| **Mix A2B1 vs LAC 127 µM, BCP 25 µM** | 72.74 | <0.0001 |
| **Mix A2B3 vs LAC 127 µM, BCP 100 µM** | 482.7 | <0.0001 |
| **Mix A3B1 vs LAC 254 µM, BCP 25 µM** | 272.6 | <0.0001 |
| **Mix A3B2 vs LAC 254 µM, BCP 50 µM** | 750.6 | <0.0001 |

### EXAMPLE 3 - IL 6, IL-8 gene expression in HMC3 cell line

To confirm the results obtained from ELISA assays, the modification in IL-6, IL-8, gene expression after the treatments with BCP and LAC alone, and their combinations were monitored using quantitative reverse transcription PCR (RT-qPCR).

As LAC and BCP alone and their combinations induce the increase in the release of IL-8 and IL-6, the expected results of this further test should be a decrease in the expression of mRNAs coding for IL-8 and IL-6.

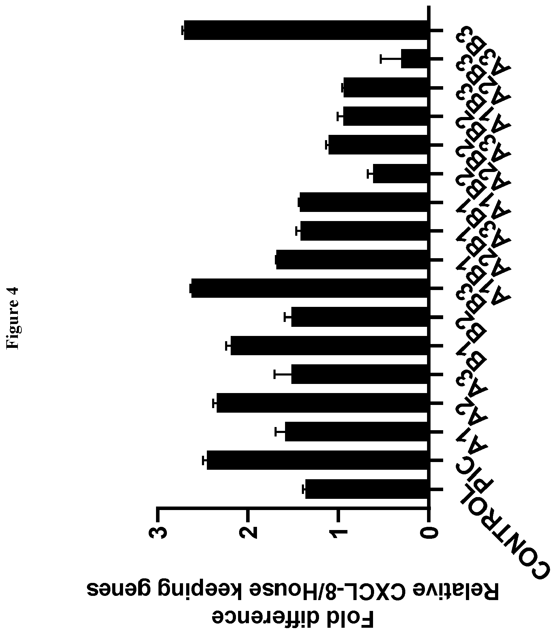

As expected, the expression of the mRNAs coding for IL-6 and IL-8 followed the same trends of the concentrations of the proteins determined with the ELISA assay. In particular, as it can be appreciated from **Figure 4****,** Mix A2B1, A1B2, A2B2, A3B2, A2B3 and A1B3 revealed a statistically significant reduction in the gene expression of IL-8 mRNA in comparison with the substances alone.

The One-way Anova statistical analysis on A1B3, A2B2, A1B2, A3B2, A2B3, and A2B 1 revealed a difference between the five groups analyzed. The Tukey test underlines a down-regulation of IL-8 mRNA when HMC3 cells are treated with BCP and LAC in combination compared with the same substances taken alone (Table 5).

**Table 5- Results of one-way Anova test (Tukey's multiple comparison) performed with BCP, LAC and their combinations on down-regulation of IL-8 mRNA in real-time PCR.**

| **Experimental model** | **Test F** | **P-value** |
|---|---|---|
| **Mix A1B2 vs LAC 63.5 mM, BCP 50 µM** | 319.8 | <0.0001 |
| **Mix A2B2 vs LAC 127 mM, BCP 50 µM** | 735.7 | <0.0001 |
| **Mix A1B3 vs LAC 63.5 mM, BCP 100 µM** | 3262 | <0.0001 |
| **Mix A2B3 vs LAC 127 mM, BCP 100 µM** | 272.3 | <0.0001 |
| **Mix A3B2 vs LAC 254 mM, BCP 50 µM** | 81.34 | <0.0001 |

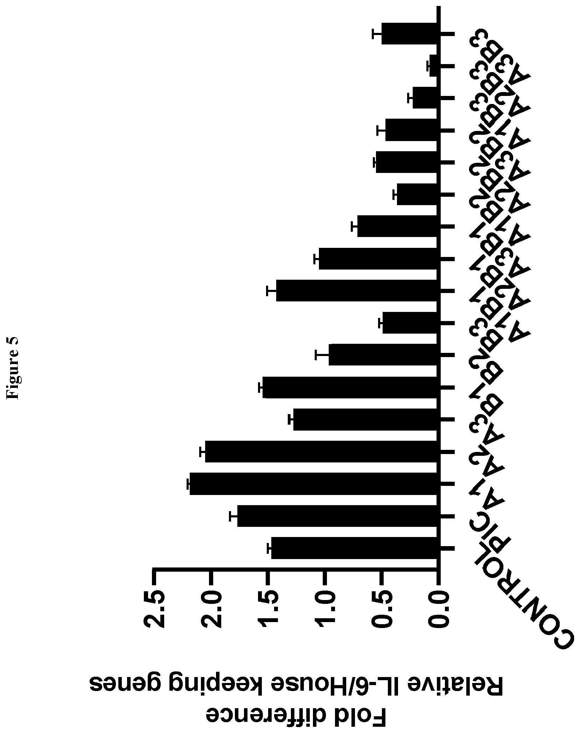

Regarding the IL-6 gene expression, Mix A2B1, A3B1, A1B2, A2B2, A3B2, A1B3 and A2B3 showed a statistically significant reduction in gene expression compared to the same substances alone (**Figure 5**).

The One-way Anova statistical analysis on A2B1, A3B1, A1B2, A2B2, A3B2, A1B3 and A2B3 revealed a difference between the five groups analyzed. The Tukey test underlines a down-regulation of Il-6 mRNA when HMC3 cells are treated with BCP and LAC in combination compared with the same substances taken alone (Table 6).

**Table 6- Results of one-way Anova test (Tukey's multiple comparison) performed with BCP, LAC and their combinations on down-regulation Il-6 mRNA in real-time PCR.**

| **Experimental model** | **Test F** | **P-value** |
|---|---|---|
| **Mix A2B1 vs LAC 127 mM, BCP 25 µM** | 438.4 | <0.0001 |
| **Mix A3B1 vs LAC 254 mM, BCP 25 µM** | 339.0 | <0.0001 |
| **Mix A1B2 vs LAC 63.5 mM, BCP 50 µM** | 503.1 | <0.0001 |
| **Mix A2B2 vs LAC 127 mM, BCP 50 µM** | 331.7 | <0.0001 |
| **Mix A3B2 vs LAC 254 mM, BCP 50 µM** | 73.91 | <0.0001 |
| **Mix A1B3 vs LAC 63.5 mM, BCP 100 µM** | 3540 | <0.0001 |
| **Mix A2B3 vs LAC 127 mM, BCP 100 µM** | 2984 | <0.0001 |

The synergistic actions of the combinations of LAC and BCP are so confirmed with two different techniques that present two different targets.

Table 7 reports the combinations of LAC and BCP which have shown synergistic activity following the ELISA and real-time PCR experiments.

**Table 7**

| **ELISA test for cytokines dosage** | **LAC (µM)** | **BCP (µM)** | **Mix** | **Approximate Ratio** |
|---|---|---|---|---|
| IL-8 | 254 | 100 | A3B3 | 2.5:1 |
| | 127 | 25 | A2B1 | 5,:1 |
| | 127 | 100 | A2B3 | 1.3:1 |
| | 254 | 25 | A3B1 | 10:1 |
| | 254 | 50 | A3B2 | 5:1 |
| IL-6 | 254 | 100 | A3B3 | 2.5:1 |
| | 127 | 25 | A2B1 | 5:1 |
| | 127 | 100 | A2B3 | 1.3:1 |
| | 254 | 25 | A3B1 | 10:1 |
| | 254 | 50 | A3B2 | 5:1 |

| **Quantification of mRNA Expression by RT-qPCR** | | | | |
|---|---|---|---|---|
| IL-8 | 63.5 | 50 | A1B2 | 1.3:1 |
| | 127 | 50 | A2B2 | 2.5:1 |
| | 63.5 | 100 | A1B3 | 0.6:1 |
| | 127 | 100 | A2B3 | 1.3:1 |
| | 254 | 50 | A3B2 | 5:1 |
| IL-6 | 127 | 25 | A2B1 | 5:1 |
| | 254 | 25 | A3B1 | 10:1 |
| | 63.5 | 50 | A1B2 | 1.3:1 |
| | 127 | 50 | A2B2 | 2.5:1 |
| | 254 | 50 | A3B2 | 5:1 |
| | 63.5 | 100 | A1B3 | 0.6:1 |
| | 127 | 100 | A2B3 | 1.3:1 |

In summary, the treatment of inflamed HMC3 cells with LAC, BCP and their combination showed that the treatment with the combinations of the two substances exerts higher anti-inflammatory activity that each substance alone, with the differences in the anti-inflammatory activities recorded with both ELISA test and qPCR assay statistically significant, showing a synergist effect between the two selected substances (LAC and BCP).

### EXAMPLE 4 - Evaluation of ROS production following treatment with BCP, LAC, alone and in combination

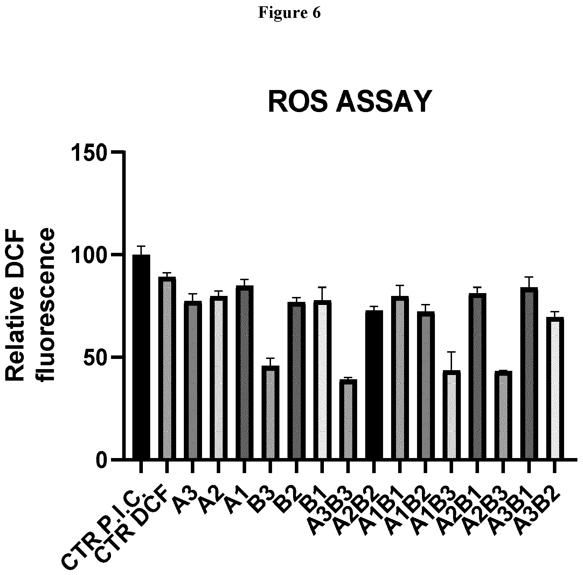

**Figure 6** shows the ROS production by HMC3 cells following the treatments reported in Table 2 with BCP and LAC, alone and in combination. In this experiment, the treatments were added to the cell culture medium within 24 h before the inflammatory insult.

All the treatments show a decrease in the production of ROS as compared to the positive control sample (P.I.C). In particular, A3B3, A1B3 and A2B3 samples revealed the greatest ROS reduction.

The following studies were performed to evaluate the whether the chronic administration of BCP-LAC exerts reliable effects on pain and neuroprotection in two rat models of NP. The studies concerned behavioural performance in vivo and biochemical and molecular pain-associated mediators by means of ex-vivo analysis.

### EXAMPLE 5 - Evaluation of behavioural performance in vivo

The effects of BCP and LAC, alone and in combination, are evaluated in vivo. The sciatic nerve chronic constriction injury (CCI), developed in rats by Bennet and Xie (Bennett and Xie, 1988, Pain 33, 87-107) and the reserpine-induced myalgia (RIM) models are used (Kiso et al., 2018, Eur J Pharmacol 827, 117-124), as in vivo rat models of neuropathic pain and fibromyalgia (nociplastic pain), respectively.

***Animals:*** Male and female Sprague Dawley rats (Charles River Laboratory, Lecco, Italy) weighing 230-250 g are used, randomly assigned to experimental groups. The animals are housed two per cage and maintained under controlled environmental conditions (22 ± 1 °C; 12:12 h light-dark cycle; food and water ad libitum) for one week before use in the experiments. The study is conducted on 480 rats (240 for CCI and 240 for RIM).

All procedures are conducted by an operator not aware of the treatment in order to avoid bias.

The results are evaluated in terms of improved behavioral scores and decreased expression of inflammatory- and pain-associated mediators.

***Statistical methods:*** All data are analyzed by 2-way repeated measures analysis of variance (ANOVA; repeated behavioral scores) or 2-way ANOVA (all other data) followed by the Tukey-Kramer or Bonferroni post-hoc. Sex is used as a covariate, since the enhanced prevalence of NP in female is known. Differences are considered significant with p value (p) <0.05.

***Experimental animals:*** two different rat models of NP are used.

***CCI*** consists in four loose ligatures around the sciatic nerve, that occlude but do not arrest epineural blood flow. It is relatively simple to perform and CCI rats manifest a pain syndrome that begins about 3 days after the nerve injury and reaches a plateau lasting between 7 and 30 days.

***RIM*** is a putative model of fibromyalgia in which muscle pressure thresholds and monoamine content in the brain and spinal cord, are reduced. Repeated administration of reserpine (1 mg/kg subcutaneously, once daily, for three consecutive days) causes long-lasting widespread muscle and cutaneous hyperalgesia that is sustained for at least 1 week in both male and female rats (Kiso et al., *supra*)*.*

Experimental groups are treated as follows:
1^{st} group: Vehicle;
2^{nd} group: LAC 140 mg/kg;
3^{rd} group: BCP 14 mg/kg;
4^{th} group: BCP 28 mg/kg;
5^{th} group: BCP 56 mg/kg;
6^{th} group: BCP 112 mg/kg;
7^{th} group: LAC 140 - BCP 14 mg/kg;
8^{th} group: LAC 140 - BCP 28 mg/kg;
9^{th} group: LAC 140 - BCP 56 mg/kg;
10^{th} group: LAC 140 - BCP 112 mg/kg.

Target molecules or vehicle are chronically administered by means of oral gavage.

### Experimental procedures:

***Rat surgery for CCI model:*** rats are subjected to the CCI technique, developed by Bennett and Xie (Bennett and Xie, *supra*)*.* The experimental procedure involves the unilateral ligation of the sciatic nerve at the high-thigh level of the right hind paw. Rats are deeply anesthetized by inhalation of a mixture of isoflurane and oxygen. The cutting area is first shaved and then, under sterile precautions, the sciatic nerve is exposed. Proximal to its trifurcation, 4 ligatures with 3-0 silk suture are tied loosely around the nerve at intervals of around 2 mm, so that the length of the treated nerve is approximately 8-10 mm, to preserve the epineural circulation. The wound is closed with three external stitches and disinfected. Sham-operation consists in anesthesia and surgery up to sciatic nerve exposure without ligation (Bellei et al., 2017 Sci Rep 7, 41723).

***Reserpine treatment for RIM model*** (Nagakura, 2022, J Pharmacol Exp Ther 381, 106-119): reserpine is administered to rats at 1 mg/kg subcutaneously, once daily, for three consecutive days.

***Behavioral assessment of pain in CCI rats:*** within few hours after the surgery, rats with sciatic nerve ligation develop spontaneous pain manifested by guarding behaviour of the operated paw and licking on the injury side. In the following days, they show signs of paw lifting and limping, excessive grooming and biting, considered as nocifensive signs indicating the presence of spontaneous pain. All these behaviors are daily videorecorded and analyzed over the entire experimental time-window.

Moreover, rats with sciatic nerve ligation exhibit signs of allodynia to mechanical stimulation assessed by Von Frey test 2 and 5 weeks after surgery, compared to sham operated rats; pain indicator signals persist for the entire duration of the experiment (Bellei et al., *supra*)*.*

***Von Frey hair test:*** mechanical sensitivity (allodynia) is assessed by Von Frey hair test, based on the responsiveness of the operated hind paw to the application of a series of von Frey filaments (Aesthesio^{®}, USA), with progressively increased stiffness (ranging from 0.008 to 300 g). Briefly, animals are placed in a plexiglass cage with a metal grid floor, allowing them to move freely. Filaments are applied to the mid-plantar surface of both the injured and non-injured hind paw. The paw sensitivity threshold is set as the minimum force required to induce a robust and immediate withdrawal of the paw. Then, the smallest filament that elicited a foot withdrawal response is considered the threshold stimulus (Bellei et al., *supra*)*.*

***Plantar test:*** thermal sensitivity (hyperalgesia) is assessed by means of Hargreaves Apparatus (Ugo Basile). A thermal stimulus, generated by a focused light source, is applied through a glass pane to the plantar surface of the hind paw of the animal, inducing withdrawal of the stimulated paw. The average reaction time is calculated as the average of 5 repeated trials, 5-minute apart (Abed et al., 2021, Res Pharm Sci 16, 250-259).

***Other behavioral assessments for both CCI and RIM rats:*** in vivo rat models of NP manifest signs of the co-morbid symptom such as induced anxiety and depression. These parameters are measured before and after interventions by means of open field test, elevated plus maze and forced-swimming test. Muscular strength and motor coordination are also measured by Rotarod test and grip test, respectively.

All rat behaviors are recorded and analyzed by advanced ANYMAZE and/or Ethovision videotracking systems for animal behavior.

### EX VIVO PROCEDURES AND ANALYSIS

***Blood collection and serum preparation for proteomic analysis:*** blood is collected retro-orbitally in vacutainer serum separation tubes and allowed to clot at room temperature for 1 h. Serum is separated by centrifugation at 2000 × g for 10 min at +4 °C. After the addition of a protease inhibitor cocktail (Sigma-Aldrich) to prevent protein enzymatic breakdown or modifications, samples are divided into aliquots and kept frozen at -80 °C until use. To reduce the complexity of serum samples before proteomic analysis, sample are treated with the ProteoPrep^{®} Immunoaffinity albumin and IgG depletion kit (Sigma-Aldrich), that specifically removes the two most abundant serum proteins, albumin and IgG. The protein content after depletion is assayed by the spectrophotometric Bradford's method, using bovine serum albumin (BSA) as standard (Bellei et al., *supra*)*.*

***Histological processing:*** At the end of the behavioral test, rats are anesthetized with isoflurane and sacrificed for histological processing. Brain, liver, spleen and kidneys are dissected out. The brains are postfixed in the same solution for 12 h and rinsed in a 30% sucrose-PBS solution for two days. The brains are frozen using dry ice, and coronal 40 µm thick sections are obtained by cryostat cutting and stored at 20°C in a glycerol-PBS solution until use. The expression of inflammatory markers is analyzed at cellular- and brain area-specific levels (Daini et al., 2021, Neurosci Lett 754, 135869; Vilella et al., 2023, Front Neurosci 17, 1097857).

***Molecular and proteomic analysis:*** Another group of rats are anesthetized with isoflurane and sacrificed for molecular and proteomic analysis; brain, liver, spleen and kidneys, as well as brain and lumbar spinal cord are dissected and fast frozen with liquid nitrogen.

***Quantitative real-time polymerase chain reaction and western blot analysis:*** after animal sacrifice, different brain regions and lumbar spinal cord are dissected out, lysed by mechanical disruption. Half of the tissues are immersed in Trizol reagent (Qiagen), homogenized following the procedure provided by the manufacturer (RNeasy Plus Mini Kit, Qiagen) and processed for quantitative PCR (qPCR). The other half are homogenized in RIPA buffer for protein extraction. Both RNAs and proteins are used for measuring the expression of inflammatory-related markers and pain-associated biomarkers (Bellei et al., *supra*)*.*

**SDS-PAGE and two-dimensional gel electrophoresis:** the proteomic analysis is performed on depleted serum samples, first by sodium dodecyl sulphate-polyacrylamide gel electrophoresis (SDS-PAGE) and then by two-dimensional gel electrophoresis (2-DE). Secondarily, selected bands are analysed by means of mass spectrometry (MS) analysis as previously described (Bellei et al., *supra*)*.*

Examples of pharmaceutical formulations are reported hereinbelow.

### EXAMPLE 6 - Sachet for oral use

A sachet is prepared having the following composition:

| Ingredient | per sachet |
|---|---|
| L-acetyl carnitine | 1.000 mg |
| Piper Nigrum extract* | 100 mg |
| Alpha lipoic acid | 600 mg |
| *Boswellia serrata* e.s. | 200 mg |
| Vitamine B6 | 2,8 mg |
| Vitamine E | 24 mg |

| | |
|---|---|
| * as beta-caryophyllene content | |

### EXAMPLE 7 - Sachet for oral use

A sachet is prepared having the following composition:

| Ingredient | per sachet |
|---|---|
| L-acetyl carnitine | 1.000 mg |
| Piper Nigrum extract* | 100 mg |
| Alpha lipoic acid | 600 mg |
| *Boswellia serrata* e.s. | 200 mg |
| Palmitoylethanolamide | 600 mg |
| Vitamine B6 | 2,8 mg |
| Vitamine E | 24 mg |

| | |
|---|---|
| * as beta-caryophyllene content | |

### EXAMPLE 8 - Tablets for Oral Use

Tablets are prepared having the following composition:

| Ingredient | per tablet |
|---|---|
| L-acetyl carnitine | 500 mg |
| Beta-caryophyllene | 100 mg |
| Microcrystalline Cellulose | 70 mg |
| Polyvinylpyrrolidone | 25 mg |
| Magnesium stearate | 5.0 mg |
| Total | 700 mg |

## Claims

1. A combination comprising L-acetyl carnitine or a pharmaceutically acceptable salt thereof, and beta-caryophyllene wherein the molar ratio is comprised between 1:2 and 20:1.

2. The combination according to claim 1, wherein the molar ratio ranges between 1:1 and 15:1.

3. The combination according to claim 2, wherein the molar ratio ranges between 2:1 and 10:1.

4. The combination according to any one of claims 1 to 3, in the form of a fixed combination or a loose combination.

5. The combination according to claim 4, in the form of a fixed combination.

6. A nutraceutical and/or food supplement composition comprising a combination according to any one of claims 1 to 5, wherein the molar ratio between L-acetyl carnitine or a pharmaceutically acceptable salt thereof, and beta-caryophyllene ranges from 1:1.5 to 12:1, and at least one physiologically acceptable excipient and/or additive.

7. A pharmaceutical composition comprising a combination according to any one of claims 1 to 5, wherein the molar ratio between L-acetyl carnitine or a pharmaceutically acceptable salt thereof, and beta-caryophyllene ranges from 1:2 to 10:1, and at least one pharmaceutically acceptable excipient and/or additive.

8. The composition according to claims 6 or 7, further comprising at least one additional active ingredient selected from antioxidants, anti-inflammatory agents and/or vitamins.

9. The composition according to claim 8, wherein the antioxidant is selected from the group comprising polyphenols, alpha-lipoic acid, vitamins, coenzyme Q10, and glutathione.

10. The composition according to claim 8, wherein polyphenols are selected from polydatin, resveratrol, luteolin, quercetin and rutin.

11. The composition according to claim 8, wherein the anti-inflammatory agents are selected from palmitoylethanolamide and *Boswellia serrata* extracts.

12. The composition according to claim 8, wherein vitamins are selected from vitamins of the B group, preferably vitamin B6 and vitamin E.

13. A combination comprising L-acetyl carnitine or a pharmaceutically acceptable salt thereof, and beta-caryophyllene or a composition thereof for use as a medicament.

14. The combination or a composition for use according to claim 13 in the prevention and/or treatment of mild, moderate to severe, acute or chronic pain and pain-correlated diseases, preferably neuropathies and/or inflammatory pain.

15. The combination or a composition for use according to claim 14, wherein the pain is selected from the group comprising pain caused by trauma, or surgical intervention such as post-operative pain; pain caused by diseases such as cancer, AIDS, myocardial infarction, renal or biliary colic, chronic inflammation and chronic neuropathies; and wherein said pain-correlated diseases are selected from the group comprising peripheral tissue injury; nerve injury associated with trauma, radiation damage, surgery, crushed limbs, amputation; cancer; non-malignant progressive diseases, such as AIDS, sickle cell anemia, hemophilia, and connective tissue diseases; non-progressive or slowly progressive diseases, such as severe osteoporosis, gout, post-herpetic neuralgia, painful polyneuropathy, reflex sympathetic dystrophy; and idiopathic syndromes, such as fibromyalgia, atypical facial pain, chronic pelvic pain of unknown etiology, and neuropathies; preferably, neuropathies in which pain is due to a trapped and/or compressed nerve, such as carpal tunnel syndrome, herniated disc, cervicobrachial pain, neck pain, low back pain.

16. A kit comprising:
a) L-acetyl carnitine or a pharmaceutically acceptable salt thereof in a first dosage unit form
b) beta-caryophyllene in a second dosage unit form;
and optionally
c) a unique packaging container for said first and second dosage forms.
